(19) Europäisches Patentamt
European Patent Office
Office européen des brevets

(11) **EP 2 420 147 B1**

(12) **EUROPEAN PATENT SPECIFICATION**

(45) Date of publication and mention
of the grant of the patent:
**17.05.2017 Bulletin 2017/20**

(51) Int Cl.:
**A23L 33/10** (2016.01)     **A23L 33/175** (2016.01)
**A23L 33/13** (2016.01)     **A23L 33/15** (2016.01)

(21) Application number: **11075190.6**

(22) Date of filing: **09.08.2011**

(54) **Nutritional supplement compositon**

Ernährungsergänzungszusammensetzung

Composition de complément nutritionnel

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **17.08.2010 EP 10380106**

(43) Date of publication of application:
**22.02.2012 Bulletin 2012/08**

(73) Proprietor: **Vitae Natural Nutrition, S.L.**
**08172 Sant Cugat del Vallès Barcelona (ES)**

(72) Inventors:
• **Suñe Negre, José Maria**
**08172 Sant Cugat del vallès (Barcelona) (ES)**
• **Roig Carreras, Manuel**
**08172 San Cugat del Vallès (Barcelona) (ES)**

• **Sarrate Arjona, Rocio**
**08172 Sant Cugat del vallès (Barcelona) (ES)**
• **Cortada Pasola, Raimon**
**08172 Sant Cugat del vallès (Barcelona) (ES)**

(74) Representative: **Torner Lasalle, Elisabet**
**Torner, Juncosa I Associats, S.L.**
**C/Gran Via de les Corts Catalanes, 669bis 1è 2o**
**08013 Barcelona (ES)**

(56) References cited:
**WO-A2-2005/006890     DE-A1- 10 326 822**
**DE-A1-102008 004 090     DE-U1-202008 006 741**
**US-A- 3 816 262     US-A1- 2003 021 772**

Remarks:
The file contains technical information submitted after the application was filed and not included in this specification

**Description**

From the recent years, several nutritional formulations are know particularly for its advantageous compositions to reduce, for example, physical and mental fatigue, to enhance activity, to improve recovery after intense activity, to promote muscle performance, to increase energy, to contribute improving antioxidant defenses (i.e., reduce oxidative stress or lipid per oxidation, conserve anti-oxidants in the serum), to enhance mood, to assist in preventing primary and secondary diseases associated with fatigue and muscle atrophy associated with inactivity, to improve nervous system (i.e., neuronal) and musculoskeletal health (i.e., increase skeletal muscle protein synthesis, increase satellite cells), and to contribute improving overall health. The above formulations are usually in the form of dietary supplement and have to be taken in addition to common alimentary programs.

For example, several formulations containing NADH are available on the market as dietary supplement.

Nicotinamide adenine dinucleotide, abbreviated $NAD^+$, is a coenzyme found all living cells. The compound is a dinucleotide, since it consists of two nucleotides joined through their phosphate groups, with one nucleotide containing an adenine base and the other containing nicotinamide.

In metabolism, $NAD^+$ is involved in redox reactions, carrying electrons from one reaction to another. The coenzyme is, therefore, found in two forms in cells: $NAD^+$, that is an oxidizing agent - it accepts electrons from other molecules and becomes reduced forming NADH, which can then be used as a reducing agent to donate electrons. These electron transfer reactions are the main function of $NAD^+$. However, it is also used in other cellular processes, notably as a substrate of enzymes that add or remove chemical groups from proteins. Because of the importance of these functions, the enzymes involved in $NAD^+$ metabolism are targets for drug discovery.

In organisms, $NAD^+$ can be synthesized from simple building-blocks (*de novo*) from the amino acids tryptophan or aspartic acid. In an alternative fashion, more complex components of the coenzymes are taken up from food as the vitamin called niacin.

NADH is directly involved in the body's cellular immune defensive system. NADH, biologically known as Coenzyme 1 (as it is the most important coenzyme, also known as Co-E1), is thus necessary for thousands of biochemical reactions within the body and is found naturally in every living cell.

As a dietary supplement NADH is currently commercialized to help boost energy and add vitality to the human body. Nutritional compositions containing NADH may also aid in combating the effects of disorders such as fibromyalgia, that is a physical disorder that causes the sensory system to become hyperaware of environmental stimuli such as pain, and to alleviate the symptoms of certain energetic disorders such as fatigue, particularly chronic fatigue syndrome, which is often related to stress and which is defined as a condition in which a patient has persistent fatigue not attributable to any other cause.

Other dietary supplement is known, comprising for example Coenzyme Q10, that is also known as ubiquinone, ubidecarenone, coenzyme Q, and abbreviated at times to $CoQ_{10}$, CoQ, Q10, or Q. This compound is a 1,4-benzoquinone, where Q refers to the quinone chemical group, and 10 refers to the number of isoprenyl chemical subunits.

This oil-soluble substance is present in most eukaryotic cells, primarily in the mitochondria. It is a component of the electron transport chain and participates in aerobic cellular respiration, generating energy in the form of ATP.

Because of its ability to transfer electrons and, therefore, act as an antioxidant, $CoQ_{10}$ is used as a dietary supplement, for example in the following applications: mitochondrial disorders, heart failure, headaches, cardiac arrest, blood pressure, periodontal disease, Parkinson's disease.

Formulations containing both NADH and CoQ10 are also known as dietary supplement because of their capacity to supply energy, miming energy production mechanisms and the antioxidant activity usually occurs in the human body.

Another product that is widely used as a dietary supplement is Vitamin C, also called ascorbic acid. It is a water-soluble nutrient easily excreted from the body when not needed. It's so critical for life that almost all mammals use their own cells to make it.

Humans vary greatly in their vitamin C requirement. It's natural for one person to need 10 times as much vitamin C as another person; and a person's age and health status can dramatically change his or her need for vitamin C.

Most forms of cardiovascular disease, joint disease, cancer, eye disease, thyroid disease, liver disease, and lung disease require special emphasis on vitamin C intake. The process of aging itself requires special attention to vitamin C. In addition to these broader categories, several specific health conditions also require special emphasis on vitamin C. These specific health conditions include, for example, asthenia, acne, Alzheimer's disease, asthma, depression, diabetes, Parkinson's disease. The beneficial effects of ascorbic acid are due to its intervention in the tyrosine metabolism, leading, at the end of the biochemical pathway the synthesis of adrenalin.

Dietary supplements typically contain vitamin C as ascorbic acid. Because vitamin C is better absorbed in the presence of flavonoids, many supplement manufacturers also include flavonoids in their formulations.

Buffered versions of vitamin C are also commonly available. These buffered forms usually combine vitamin C with minerals like calcium, magnesium, or potassium. Buffered vitamin C may be helpful for individuals who have stomach sensitivity, or who are taking higher doses of the supplement.

Also widely available formulations have a metabolite complex form of vitamin C, where the ascorbic acid is combined with several of its naturally occurring metabolites including dehydroascorbate, threonate, aldonic acids and also CoQ10.

Compositions containing NADH and Vitamin C are also known as dietary supplement, indicated as adjuvant for fatigue situations as well as an excellent antioxidant.

D1 (WO 2005/006890) discloses foods, beverages, condiments, spices and salad dressings with specialized supplements. Particularly, D1 discloses weight loss supplements (point 6, page 18), **enhanced brain and nerve function supplements (point 7, page 25),** enhanced supplements designed to assist or improve liver function and/or prevent liver damage and/or disease and address other maladies associated with alcohol consumption (point 8, page 36) and many other kinds of supplements.

WO 2005/006890 discloses a very long list of components (from component A to component M), which, all together, would be able to provide the incorporation in a food, beverage, condiment, spice or salad dressing of a formulation for enhancing and maintaining neurological functions and for normalizing impaired or deteriorating neurological function (page 25, lines 22-25). CoQ10, phosphatidyl serine, vitamin C and NADH are cited in table X (pages 34 to 36) as 4 out of 47 other components all together listed and described as active ingredients of a preferred embodiment of the orally administered brain or nerve enhancement supplement according to WO 2005/006890.

DE 10326822 discloses a composition comprising NADH, L-carnitine, CoQ10, L-carnosine, ethylendicarboxylic acid, Vitamin C and bioflavonoids. All the disclosed components are essential for the activity of the supplement preparation therein disclosed.

An object of the present invention is to provide new compositions comprising different compatible active ingredients, to be used as dietary supplements for humans.

Another object of the present invention is to provide new formulations including different compatible active ingredients, to be used as dietary supplement and nutritional products with common drugs and medicaments avoiding unexpected side effects.

Still another object of the present invention is to provide an improved manufacturing process for the bulk allowing the tablet presentation and still maintaining the chemical-physical characteristics, as well as the homogeneity of the active ingredients in the formulation.

These and also other objects are achieved by a new dietary supplement composition consisting of, as active ingredients:

   A.

   - NADH and/or NAD$^+$
   - wet granulated Coenzyme Q10
   - Vitamin C (Ascorbic Acid)
   - Serine and/or Phosphoserine

   B.

   - compounds (pharmacological inactive ingredients) added to the formulation to improve characteristics of the bulk mixture selected as, lactose, dibasic calcium phosphate, sucrose, corn (maize) starch, microcrystalline cellulose, povidone polyvinylpyrrolidone, modified cellulose, magnesium stearate and calcium stearate,

and

   C.

   - pharmacological inactive additives and excipients selected as hydroxypropylmethyl cellulose gel, maltodextrine and Stark 1500.

The formulation comprises further, as component C, usual excipients and ingredients needed as a dietary supplement.

A dietary supplement, also known as food supplement or nutritional supplement, is a preparation intended to supplement the diet and provide nutrients, such as vitamins, minerals, fiber, fatty acids, or amino acids, that may be missed or may not be consumed enough with the diet. Some countries define dietary supplements as foods, while in others they are considered as drugs or natural health products.

According to the present invention, each of the active ingredient listed above (A), is essential for the aim claimed.

Serine (abbreviated as Ser or S) is an organic compound with the formula $HO_2CCH(NH_2)CH_2OH$. It is one of the natural proteinogenic amino acids synthesis and is also important for the metabolism of the purines and pyrimidines biosinthesis. It is the precursor of several amino acids including glycine & cysteine, and tryptophan in bacteria. Also it is the precursor of several other metabolites, including sphingolipids and folate, which is the principal donor of one-carbon fragments in biosynthesis.

While serine turns into glycine, it originates a kind of "active formaldehyde". This reaction is one of the most important reactions producing C1 fragments (for example methyl, formaldehyde and formiate groups).

Serine is also a biological precursor of cholamine and plays an important role in the metabolism of carbohydrates, mainly through two different pathways. Serine plays an important role in the catalytic function of many enzymes. It has been demonstrated that appears in the active sites of chymotrypsin, trypsin, and many other enzymes.

As a constituent (residue) of proteins, its side chain can undergo *O*-linked glycosylation, which may be functionally related to diabetes. It is one of three amino acid residues that are commonly phosphorylated by kinases during the cell signal in eukaryotes. Phosphorylated serine co products are often named as phosphoserine. Serine proteases are a common type of protease.

Serine is a water soluble compound but not soluble in ethanol.

The formulation according to the present invention shows a surprising effect as a dietary (nutritional) supplement due to a synergic effect of its essential active ingredients and, particularly because of the addition of serine and/or phosphoserine to the other components, NADH and/or NAD$^+$, Coenzyme Q10 and Vitamin C (Ascorbic Acid).

In fact, it has been observed that the composition according to the invention can positively influence some main natural occurring redox processes, transferring electrons to enzymes that act as biocatalysts in these reactions. For example, one of these reactions is dopamine hydroxylation to form noradrenalin and its prosecution process to give adrenaline as the final product.

The sympathomimetic adrenaline effect is significant for example during emotional crisis or when an alert situation is needed or to prepare the body to an emergency situation. In fact, adrenaline acts as a stimulant of the sympathic system and is able to produce positive effects to the main central nervous system.

It has to be noticed that it is not feasible to administer adrenalin orally, because it undergoes oxidation and degradation/conjugation during the gastrointestinal transit and in the liver.

Therefore, it has been found that a supplement dietary formulation including serine and/or phosphoserine, NADH and/or NAD$^+$, Coenzyme Q10 and Vitamin C (Ascorbic Acid) as the active and essential ingredients, can surprisingly and significantly promote the natural production of adrenaline in the human body, while avoiding undesired side effects.

Therefore, according to the present invention, the composition comprising serine and/or phosphoserine, NADH and/or NAD$^+$, Coenzyme Q10 and Vitamin C (Ascorbic Acid) as the active and essential ingredients, is advantageously used as a dietary supplement in cases where there is the need of improving and/or recovering energy, for example during a disease recovery. In this case, for example, it has been observed that the composition acts as a stimulator in case of weakness, fatigue, sleepiness and others similar side effects

For example, the formulation can be advantageously used as adjuvant dietary supplement for those persons who need particular attention, such as professional drivers (cars, trucks and similar).

Another related utilization of the composition is the reduction of the sleepiness caused by some drugs, thus resolving a very widespread problem. For example, those forced to take antihistaminic drugs suffer from very relevant problems of sleepiness. On the market, already exists associations between antihistaminic drugs and stimulating ones to overcome the side effect. However the exciting action of the stimulating drug, is difficult to be correctly dosed and can cause very often worse side effects like keeping the patient awake too much time and thus causing an associated fatigue syndrome.

The association of an antihistaminic drug with a dietary supplement according to the present invention, would allow to physiologically modulate the stimulating effect and thus avoiding an over response and the presence of additional side effects.

According to the current invention, the wording "association" or "combination" means that the formulation according to the invention and the antihistaminic or other already existing drugs (which have sleepiness as major side effect) are administered together but in separate forms as different formulations, or in different moments also in separate forms and different formulations or in a single form or formulation, while both existing in separate and different entities within the formulation.

A product able to induce a natural and endogenous stimulator effect, which can effectively contrast certain drugs sleepiness side effect is a long felt need, particularly for those professionals who must drive cars, trucks, trains and airplanes

In Spain as an example, about 5% of traffic accidents are related with drugs intake. At least around 17% of drivers declare to be on drug treatment, from them it is estimated that at least 26,4% of these involve drugs with predicted sleepiness side effects. This problem is overwhelmed by the fact that 76,5% of the total patients declare not to have been advised on the potential drugs side effects in relation to their capability to drive. Another added problem is because most of the patients taking drugs with side effects related to sleepiness, do not visit a doctor before and during the drug

**EP 2 420 147 B1**

intake, and thus are not warned about possible adverse side effects.

The composition according to the present invention represents a great improvement in the dietary supplements. In fact, the surprising effect of reducing sleepiness while still inducing a better energy feeling, result in a innovative nutritional supplement compared to existing products, which are active on the energy levels, but do not have any effect against the sleepiness. It has to be noticed, as already wrote above, that the composition, is able to induce a positive response of the human body with respect to sleepiness, without causing adverse side effect as fatigue or similar, as the response of the body is a kind of "natural" and "endogenous" reaction not caused by additional drugs.

The suggested use of this dietary supplement is therefore indicated for all cases where an increase/restoration of the energy body is required, together with the attention recovery levels both affected by several causes, among which, for example, the intake with adverse side effects related to sleepiness.

Always according to the invention, some examples of compositions containing serine and/or phosphoserine, NADH and/or NAD+, Coenzyme Q10 and Vitamin C (Ascorbic Acid) as active ingredients, are below indicated in Table 1.

**TABLE 1**

| Active ingredient | Formula 1 | Formula 2 | Formula 3 | Formula 4 |
|---|---|---|---|---|
| NAD+ or NADH | 10mg | 5-10mg | 10mg | 5-20mg |
| CoQ10 | 100mg | 60-100mg | 90mg | 200mg |
| Serine or fosfatidilserine | 40mg | 40mg | 50mg | 80mg |
| Vitamin e C (Ascorbic acid) | 90mg | 140mg | 100mg | 150mg |
| Excipie nts q.s.p | Aprox 250-500mg | Aprox 300mg | Aprox 350mg | Aprox 500mg |

It is intended that the above formulations are given only as examples of dietary supplement compositions according to the invention and they do not in any way, represent a limitation of the scope of protection.

The dietary supplement compositions according to the invention can be formulated in all forms suitable for oral administration, for example tablets, film-coated tablets, sublingual tablets, dispersible tablet, oral dispersible tablets, sachets, capsules, powder, pellets and micro-pellets, soluble effervescent granules, being tablets the preferred formulation form.

Always according to the invention, the dietary supplement compositions can also be formulated for rectal, nasal administration, or any other suitable administration form.

According to the present invention, a manufacturing process is provided for the preparation of the dietary supplement composition, particularly suitable when the composition must be formulated in tablet form.

In the tablet compressing process, it is important that all ingredients are fairly dry, in powder or granulate have to be somewhat uniform in particle size, and with good flowing characteristics. The powder mix can segregate during manufacturing /packaging operations because of the different ingredient densities, causing a poor content uniformity and this has to ensure the dose of active ingredients with each tablet.

Most formulations include excipients, pharmacological inactive ingredients like *binders, which* are added to the formulation to improve the aggregation of the tablet ingredients and give its strength. A wide range of binders exists, like lactose, dibasic calcium phosphate, sucrose, corn (maize) starch, microcrystalline cellulose, povidone polyvinylpyrrolidone and modified cellulose (i.e. Hydroxypropyl methylcellulose and hydroxyethylcellulose).

Often, an ingredient is also needed to act as a *disintegrating agent* to aid tablet dispersion once swallowed, releasing the active ingredients for its body absorption. Some binders, such as starch and cellulose, are also excellent disintegrants.

Small amounts of *lubricants* are usually added, as well. The most common one is magnesium stearate and calcium stearate.

In the tablet pressing process, the main target and objective is to ensure that the appropriate amount of active ingredients is present in each tablet. Hence, all the ingredients should be well-mixed to have a homogeneous formulation. If this is not possible with a simple blending process, then the ingredients will have to be granulated prior to the compression step to assure a good distribution of the active compounds in the final tablet. Powders with good mixing characteristics do not require previous granulation and can be compressed into tablets through a simple direct compression.

Two basic techniques are used to granulate powders for tablet compression: wet granulation and dry granulation.

Wet granulation is a process of using a liquid binder to lightly agglomerate the powder mixture. The amount of liquid added to the granulation has to be properly dosed, because an over-wetting could originate the granules to be too hard and an under-wetting could cause a too soft and friable tablet. The aqueous binder solutions have the advantage of being safer during manufacturing but may not be suitable for drugs which are degraded by hydrolysis.

• Procedure

◦ Step 1: The active ingredient and excipients are weighed and mixed.
◦ Step 2: The wet granulate is prepared by adding the liquid binder-adhesive to the powder blend and mixing thoroughly. Examples of binders/adhesives include aqueous preparations like cornstarch, natural gums (such as acacia), cellulose derivatives (such as methyl cellulose), gelatin, and povidone.
◦ Step 3: Screening the damp mass through a mesh to form pellets or granules.
◦ Step 4: Drying the granulation. A conventional tray-dryer or fluid-bed dryer are most commonly used.
◦ Step 5: After the granules are dried, they have to be passed through a smaller size sieve (than in the wet mass) to create granules of uniform size.

Whatever process is used to make the tablet bulk blend, the process of compressing is the same: First, the powder is filled into the under die. The mass of powder is determined by the position of the lower punch in the die, the cross-sectional area of the die, and the powder density. At this stage, adjustments to the tablet weight are normally made by re-adjusting the lower punch. After the under die is filled, the upper punch is lowered into the die and the powder is compressed to a porosity of between 5 and 20%. The compression can take place in one or two stages (main compression, and, sometimes, precompression or tamping) at a speed of 50-500 msec/strock. Finally, the upper punch is pulled up and out of the die (decompression), and the tablet is ejected from the die by lifting the lower punch until its upper surface is flush with the top face of the die. This is the process simply repeated same times as tablet manufactured

During the development process with the above summarized known techniques, it has been observed that Coenzyme Q10, (which is characterized by a very low melting point (48°C)) was difficult to manage and easy to melt during the process, being very difficult to formulate together with the other active ingredients, according to the invention.

It is possible to indicate a kind of "adherence by melting index" $I_{afx}$ which is defined as follows:

$$I_{axf} = C\% \times T_p\% / P_f$$

where C% is active ingredient (CoQ10) concentration in the above mentioned formula, $T_p\%$ is the particles concentration, when their size is lower than 50 μm and $P_f$ is CoQ10 melting point.

It is clear that adherence by melting is directly related to CoQ10 concentration and to particles concentration when they have a particles size lower than 50 μm as well as inversely related to its melting point.

According to the present invention:

$$I_{afx} = 10 \times 19{,}36 / 48 = 4{,}03$$

Taking into consideration, as acceptable limit:

$$I_{afx} \leq 1 \text{ y } P_f > 40$$

The obtained value of 4,03 means that the adherence probability is very high.

The (at least partial) melt of Coenzyme Q10 is probably due to the high forces necessary to carry out the compression process increasing the temperature above the melting temperature of Coenzyme Q10, at least in some parts of the mixture. As a consequence, the powder mixture sticks in the die during the tablet compression, causing a significant weight of the mixture to be formulated as well as having tablets with the correct weight target, shape and homogeneous composition.

The above mentioned problem has been tried to be solved using a wet granulation technique, starting from an excipient mixtures able to form a net, within Coenzyme Q10 stay disperse and thus avoiding a compact mixture that would not help.

Most used excipients are cellulose (various types), polyols (various polymers), poloxamers, carbomers, maltodextrine, Stark 1500® (pre gelatinized starch), Si anhydride, soy lecithin, talcum powder, magnesium stearate, sodium stearilfumarate, croscarmellosa.

However, the obtained results were not completely satisfactory, as the final product still stuck on the die, giving the above mentioned problems.

According to the present invention, the problem was solved with a wet granulation of Coenzyme Q10 where the excipients were selected as hydroxypropylmethyl cellulose gel (having film forming properties), maltodextrine (giving the desired water solubility) and Stark 1500® (Starch 1500® is a partially pre gelatinized maize starch commercialized as pharmaceutical excipient able to combine several properties in a single product. It acts with multiple functions, as a binder, disintegrant, and flow-aid while having lubricant properties.It is commercialized by Colorcon Europe).

The granulate obtained according to the present invention is characterized by a film forming, water soluble and gelled matrix, containing Coenzyme Q10 dispersed and trapped on the special net formed by the excipients.

Following the described invention and these particular excipients, finally was possible to carry out a process for the preparation of the composition according to the invention that does not cause melting of Coenzyme Q10 and the consequent stick of the powder mixture to the die.

Therefore it has to be noted that, in addition to the problems related to CoQ10 melting, also NADH can suffer from partial or total degradation at acid values of pH, while ascorbic acid tends to have a sort of degradation at alkaline pH values.

Furthermore, solutions (but also powder forms) of ascorbic acid could react with serine according to Maillard reaction, in determined high temperature conditions.

According to this process, it is therefore possible to prepare the dietary composition according to the present invention and press it into tablets, avoiding the problems caused by the compression of Coenzyme Q10 and its melting during tablet compression

The process is characterized by the presence of Coenzyme Q10 in amounts comprised between 30 mg and 100 mg, starting from a mixture of hydroxypropylmethyl cellulose in the gel form.

Another possibility is to mix maltodextrin and pre gelatinized starch into which granulated hydroxypropylmethyl cellulose containing Coenzyme Q10 is subsequently added, the resulting tablets containing between 30 mg and 100 mg of CoQ10, between 50 mg and 100 mg of ascorbic acid (Vitamin C), between 20 mg and 80 mg of serine and between 5 mg and 20 mg of NADH.

As an alternative manufacturing process of the bulk mixture, it is also feasible to prepare a mixture of maltodextrin and pre gelatinized starch where Coenzyme Q10 is dispersed and subsequently granulated and to add it another mixture comprising ascorbic acid, NADH and serine.

EXAMPLE 1

Process for the production of 1000 tablets.

Several steps are necessary:

1.1. GRANULATION

I- Sieve through a 0,8 mm mesh the following components:

| | |
|---|---|
| CoQ10 | 100 g |
| Maltodextrine | 100 g |
| Starch 1500® | 240 g |
| Sodium Laurilsulfate | 4 g (humidifying agent, allows disgregation) |

**II-** Mixing all components according to point (I) as above into a biconic mixer for 20 minutes.

**III-** Preparing 165 ml of an aglutinant solution comprising HPMC at 4 % (6,60 g).

**IV-** Put into a double Z mixer (double sigma) the solution obtained according to point (III) above and mix it with the mixture obtained according to point (II) above, for about 10 minutes.

**V-** Put the mass obtained according to point (IV) above into a wet rotating pellettizer (IV) with a cylindrical sieve of 3 mm.

**VI-** Dry the granulated obtained according to point (V) above in the heater at 40 °C for 48 hours and pass through an oscillating pelletizing sieve of 1 mm.

In this way, it is possible to obtain a granulated compound where CoQ10 is trapped into a matrix formed by three different components, i.e. HPMC which, thanks to its gel forming and film forming characteristics, is able to fix maltodextrin (water soluble) and Starch 1500® (in a pre gelatinized form) to the CoQ10 core. As a consequence, CoQ10 is not directly contacting the dies, at the same time resulting in a higher melting point value and a higher particle size. Then, $I_{axf}$ value decreases and it is therefore possible to compress it without causing its adhesion to the die.

**VII-** sieve through a 0,8 mm sieve the following components:

| | |
|---|---|
| ascorbic acid as sodium ascorbate: | 90 g |
| NADH | 10 g |
| Sodium citrate bibasic (pH regulator): | 1,5 g |
| Sodium citrate tribasic (pH regulator): | 4,5 g |
| Croscarmellose (disintegrating agent): | 50 g |
| Vivapur 102 (celulose, binder): | 270,70 g |
| Aerosil ($SiO_2$ colloidal): | 4 g |
| Talcum powder: | 45 g |
| Sodium Laurilsulphate: | 5 g |

**VIII-**

Mixing all components obtained according to above step (VII) into a biconic mixer for 20 minutes.

According to the process of the invention, the introduction of ascorbic acid as sodium salt exerts a protection action from possible hydrolysis and from the acid action that could affect NADH. Always according to the invention, the introduction in the mixture of a pH regulator (in the form of citrates) avoids pH values alteration due, for example, to humidity traces as well as allows to keep pH = 6 levels, thus avoiding the possible ascorbic acid degradation.

**IX-** Serine is mixed with a portion of magnesium stearate (2g) during 10 minutes, then the mixture is sieved through a 0,8 mm sieve and added to the mixture obtained according to point (VIII) above, putting into into a biconic mixer and mixing for 15 minutes.

In this way, serine is protected against a direct contact with sodium ascorbate, thus avoiding the Maillard reaction, taking into consideration that sodium dry form of Vitamin C is less reactive.

**X.** Finally and in the same mixer according to step (IX) above, magnesium stearate is added and the resulting composition is mixed for 3 minutes.

## 1.2. TABLET

**XI-** The mixture obtained according to point (X) above, is put in a rotating machine provided with dies of 19,5 mm x 9 mm with a toughness from 120 a 140 N and a weight of 980 mg.

No adherences are observed.

**XII-** The tablets obtained according to point (XI) above, are covered by a sealing layer formed by Sepifilm (84 g), Kollidon VA 64 (36 g), water (700 g) until a weight increase of 3 % is observed. (covering drum Glatt)

With this kind of layer, the coverage of the enteric layer is made easier. The conditions of this sealing are very strict:

with reference to the processing temperature:

a- Air entrance from 40 °C to 44 °C Air exit from 30 °C to 35 °C
b- drum speed must be very slow: position between 2 and 3
c- feed speed must be, as a consequence, very low.

According to the above conditions, it is therefore possible to avoid CoQ10 melting and also avoid the friability of the tablets.

Therefore, the enteric cover will adhere avoiding the partial detach of the layer.

**XIII-** On the tablets obtained according to point (XII) above, the enteric covering is carried out, in order to protect NADH from the gastric acids with an acid pH.

The covering is carried out using the same covering drum used for the preparation of the sealing layer, in the same conditions used for said layer.

The following components are used:

| | |
|---|---|
| Eudragit L30 D55 | 511,78 g |
| Triethyl citrate | 18,39 g |
| NaOH 1 N | 51,18 g |
| Deionized water | 64,85 g |
| $TiO_2$ | 5,25 g |

(continued)

Talcum powder      39,53 g

From this mixture, in aqueous dispersion, it is possible to recover until 15% by weight of tablets.

**Claims**

1.  Dietary supplement composition formulated in tablet form, consisting of:

    A.

        - NADH and/or NAD$^+$
        - wet granulated Coenzyme Q10
        - Vitamin C (Ascorbic Acid)
        - Serine and/or Phosphoserine

    B.

        - compounds (pharmacological inactive ingredients) added to the formulation to improve characteristics of the bulk mixture selected as, lactose, dibasic calcium phosphate, sucrose, corn (maize) starch, microcrystalline cellulose, povidone polyvinylpyrrolidone, modified cellulose, magnesium stearate and calcium stearate,

    and

    C.

        - pharmacological inactive additives and excipients selected as hydroxypropylmethyl cellulose gel, maltodextrine and partially pre-gelatinised maize starch.

2.  Dietary supplement formulated in tablet form according to claim 1, **characterized in that** said component A consists of:

        • NADH and/or NAD$^+$ from 5 mg to 20 mg
        • Coenzyme Q10 from 50 mg to 200 mg
        • Vitamin C (Ascorbic Acid) from 50 mg to 500 mg
        • Serine and/or Phosphoserine from 10 mg to 200 mg

    as active ingredients
    while said component B consists of pharmacological inactive ingredients added to the formulation to improve characteristics of the bulk mixture and said component C consists of pharmacological inactive additives and excipients.

3.  Dietary supplement formulated in tablet formaccording to claim 2, **characterized in that** said component A consists of:

        • NADH and/or NAD$^+$ 10 mg to 15 mg
        • Coenzyme Q10 100 mg to 150 mg
        • Vitamin C (Ascorbic Acid) 200 mg to 300 mg
        • Serine and/or Phosphoserine 50 mg to 100 mg

    as active ingredients in addition to said component B which consists of pharmacological inactive ingredients added to the formulation to improve characteristics of the bulk mixture and said component C which consists of pharmacological inactive additives and excipients.

4.  Use of the composition according to claim 1 as a dietary supplement.

5. Composition according to claim 1 **characterized in that** it is associated/combined to at least a different drug which has sleepiness as a major side effect.

6. Composition according to claim 5, **characterized in that** said drug is an antihistaminic drug.

7. Process for the preparation of the composition of claim 1, wherein said composition is formulated in tablet form and wherein said process is a tablet compressing process where all ingredients are fairly dry, in powder or granulate, comprising the following steps: the powder is filled into the under die, the powder is compressed to a porosity of between 5 and 20%, after decompression the tablet is ejected from the die, **characterized in that** Coenzyme Q10 is wet granulated with at least one the following excipients: hydroxypropylmethyl cellulose in gel form, maltodextrin, pre gelatinized starch.

8. Process according to claim 7, **characterized in that** Coenzyme Q10 is wet granulated with hydroxypropylmethyl cellulose in gel form.

9. Process according to claim 7, **characterized in that** Coenzyme Q10 is wet granulated with maltodextrin, pre gelatinized starch.

10. Process according to claim 7, suitable for the bulk preparation of the composition of claim 1 for tablet compression.

**Patentansprüche**

1. Ernährungsergänzungszusammensetzung formuliert in Tablettenform, bestehend aus:

   A.

   - NADH und/oder NAD$^+$
   - feuchtgranuliertem Coenzym Q10
   - Vitamin C (Ascorbinsäure)
   - Serin und/oder Phosphoserin

   B.

   - Verbindungen (pharmakologischen inaktiven Bestandteilen), welche der Formulierung zugesetzt werden, um die Merkmale der Schüttgutmischung zu verbessern, ausgewählt aus Lactose, zweibasischem Calciumphosphat, Saccharose, Maisstärke, mikrokristalliner Cellulose, Povidon/Polyvinylpyrrolidon, modifizierter Cellulose, Magnesiumstearat und Calciumstearat, und

   C.

   - pharmakologischen inaktiven Additiven und Hilfsstoffen, ausgewählt aus Hydroxypropylmethylcellulosegel, Maltodextrin und teilweise vorgelatinierter Maisstärke.

2. Ernährungsergänzung formuliert in Tablettenform nach Anspruch 1, **dadurch gekennzeichnet, dass** die genannte Komponente A aus:

   • von 5 mg bis 20 mg NADH und/oder NAD$^+$
   • von 50 mg bis 200 mg Coenzym Q10
   • von 50 mg bis 500 mg Vitamin C (Ascorbinsäure)
   • von 10 mg bis 200 mg Serin und/oder Phosphoserin

   als Wirkstoffe besteht,
   während die genannte Komponente B aus pharmakologischen inaktiven Bestandteilen besteht, welche der Formulierung zugesetzt werden, um die Merkmale der Schüttgutmischung zu verbessern, und die genannte Komponente C aus pharmakologischen inaktiven Additiven und Hilfsstoffen besteht.

3. Ernährungsergänzung formuliert in Tablettenform nach Anspruch 2, **dadurch gekennzeichnet, dass** die genannte Komponente A aus:

- 10 mg bis 15 mg NADH und/oder NAD$^+$
- 100 mg bis 150 mg Coenzym Q10
- 200 mg bis 300 mg Vitamin C (Ascorbinsäure)
- 50 mg bis 100 mg Serin und/oder Phosphoserin

als Wirkstoffe besteht, zusätzlich zur Komponente B, welche aus pharmakologischen inaktiven Bestandteilen besteht, welche der Formulierung zugesetzt werden, um die Merkmale der Schüttgutmischung zu verbessern, und zur genannten Komponente C, welche aus pharmakologischen inaktiven Additiven und Hilfsstoffen besteht.

4. Verwendung der Zusammensetzung nach Anspruch 1 als Ernährungsergänzung.

5. Zusammensetzung nach Anspruch 1, **dadurch gekennzeichnet, dass** sie mit mindestens einem unterschiedlichen Arzneimittel assoziiert/kombiniert ist, welches Schläfrigkeit als hauptsächliche Nebenwirkung aufweist.

6. Zusammensetzung nach Anspruch 5, **dadurch gekennzeichnet, dass** das genannte Arzneimittel ein antihistaminisches Arzneimittel ist.

7. Verfahren zum Zubereiten der Zusammensetzung nach Anspruch 1, wobei die genannte Zusammensetzung in Tablettenform formuliert wird und wobei das genannte Verfahren ein Tablettenkompressionsverfahren ist, in welchem alle Bestandteile ziemlich trocken sind, in Pulverform oder als Granulate, umfassend die folgenden Schritte: das Pulver wird in die untere Pressform gefüllt, das Pulver wird bis zu einer Porosität zwischen 5 und 20% gepresst, nach der Dekompression wird die Tablette aus der Pressform abgetrennt, **dadurch gekennzeichnet, dass** das Coenzym Q10 mit mindestens einem der folgenden Hilfsstoffen feuchtgranuliert wird: Hydroxypropylmethylcellulose in Gelform, Maltodextrin, vorgelatinierter Stärke.

8. Verfahren nach Anspruch 7, **dadurch gekennzeichnet, dass** Coenzym Q10 mit Hydroxypropylmethylcellulose in Gelform feuchtgranuliert wird.

9. Verfahren nach Anspruch 7, **dadurch gekennzeichnet, dass** Coenzym Q10 mit Maltodextrin, vorgelatinierter Stärke feuchtgranuliert wird.

10. Verfahren nach Anspruch 7, geeignet für die Schüttgutzubereitung der Zusammensetzung nach Anspruch 1 zur Tablettenkompression.

**Revendications**

1. Composition d'un complément alimentaire en forme de comprimé, consistant en

A.

- NADH et/ou NAD$^+$
- Coenzyme Q10 granulé humide
- Vitamine C (Acide ascorbique)
- Sérine et/ou Phosphosérine

B.

- composés (ingrédients pharmacologiques inactifs) ajoutés à la formule pour perfectionner les caractéristiques du mélange en vrac choisis tels que lactose, phosphate de calcium dibasique, sucrose, amidon de maïs, cellulose microcristalline, povidone polyvynylpyrrolidone, cellulose modifiée, stéarate de magnésium et stéarate de calcium, et

C. additifs et excipients pharmacologiques inactifs choisis tels que gel de cellulose d'hydroxypropylméthyle, maltodextrine et amidon de maïs partiellement prégélatinisé.

**2.** Complément alimentaire en forme de comprimé conformément à la revendication 1, **caractérisé en ce que** ce composant A consiste en :

- NADH et/ou NAD$^+$, de 5 mg à 20 mg
- Coenzyme Q10, de 50 mg à 200 mg
- Vitamine C (Acide ascorbique), de 50 mg à 500 mg
- Sérine et/ou Phosphosérine, de 10 mg à 200 mg

comme ingrédients actifs
tandis que ce composant B consiste en des ingrédients pharmacologiques inactifs ajoutés à la formule pour perfectionner les caractéristiques du mélange en vrac et ce composant C consiste en des additifs et des excipients pharmacologiques inactifs.

**3.** Complément alimentaire en forme de comprimé conformément à la revendication 2 **caractérisé en ce que** ce composant A consiste en :

- NADH et/ou NAD$^+$, de 10 mg à 15 mg
- Coenzyme Q10, de 100 mg à 150 mg
- Vitamine C (Acide ascorbique), de 200 mg à 300 mg
- Sérine et/ou Phosphosérine, de 50 mg à 100 mg

comme ingrédients actifs en plus de ce composant B qui consiste en des ingrédients pharmacologiques inactifs ajoutés à la formule pour perfectionner les caractéristiques du mélange en vrac et ce composant C qui consiste en des additifs et des excipients pharmacologiques inactifs.

**4.** Utilisation du composé conformément à la revendication 1 comme complément alimentaire.

**5.** Composition conformément à la revendication 1 **caractérisée en ce qu'**elle est associée/combinée à au moins un médicament différent dont un effet secondaire principal est la somnolence.

**6.** Composition conformément à la revendication 5, **caractérisée en ce que** ce médicament est un médicament antihistaminique.

**7.** Méthode pour la préparation de la composition de la revendication 1, dans laquelle cette composition est formulée en forme de comprimé et dans laquelle cette méthode est une méthode de compression de comprimé où tous les ingrédients sont sensiblement secs, en poudre ou en granulé, comportant les étapes suivantes: la poudre est remplie dans l'emporte-pièce, la poudre est comprimée à une porosité de 5 à 20%, après la décompression, le comprimé est éjecté de l'emporte-pièce, **caractérisé en ce que** le Coenzyme Q10 est granulé humide avec au moins un des excipients suivants: cellulose d'hydroxypropylméthyle en forme de gel, maltodrexine, amidon prégélatinisé.

**8.** Méthode conformément à la revendication 7, **caractérisée en ce que** le Coenzyme Q10 est granulé humide avec de la cellulose d'hydroxypropylméthylène en forme de gel.

**9.** Méthode conformément à la revendication 7, **caractérisée en ce que** le Coenzyme Q10 est granulé humide avec de la maltodrexine, amidon prégélatinisé.

**10.** Méthode conformément à la revendication 7, appropriée pour la préparation en vrac de la composition de la revendication 1 pour la compression d'un comprimé.

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2005006890 A

- DE 10326822